# EUROPEAN PATENT SPECIFICATION

(11) **EP 4 072 618 B1**
(45) Date of publication and mention of the grant of the patent: **13.05.2026**
(21) Application number: 20817050.6
(22) Date of filing: 08.12.2020
(51) Int. Cl.: A61M 5/142, A61M 5/168, A61M 5/178, A61M 5/44

(54) **DRUG DELIVERY DEVICE, SYSTEM COMPRISING A DRUG DELIVERY DEVICE, AND ASSOCIATED METHOD**
ARZNEIMITTELABGABEVORRICHTUNG, SYSTEM MIT EINER ARZNEIMITTELABGABEVORRICHTUNG UND ZUGEHÖRIGES VERFAHREN
DISPOSITIF D'ADMINISTRATION DE MÉDICAMENTS, SYSTÈME COMPRENANT UN DISPOSITIF D'ADMINISTRATION DE MÉDICAMENTS ET PROCÉDÉ ASSOCIÉ

(30) Priority: 09.12.2019 EP 19306598
(43) Date of publication of application: 19.10.2022
(73) Proprietor: SANOFI, 75017 Paris (FR)
(72) Inventor: BLANCKE, Stefan, 65926 Frankfurt am Main (DE); JUGL, Michael, 65926 Frankfurt am Main / DE (DE)
(74) Representative: Schmidt, Christian
(86) International application number: PCT/EP2020/085119
(87) International publication number: WO 2021/116120

(56) References cited:
- WO-A1-2016/041872
- WO-A1-2016/120207
- WO-A2-2008/105958
- WO-A2-2017/090019
- GB-A- 2 490 721

## Description

### Background

The present disclosure relates to a drug delivery device, a system with a drug delivery device, and a method relating to a drug delivery device, e.g. a method for evaluating a drug delivery device, in particular an operation of the drug delivery device.

Drug delivery devices are usually used by users such as patients and/or caregivers or other medically trained or untrained people to administer drugs from a drug container into a body such as a human body.

Examples of such drug delivery devices can be found in WO 2016/041872 A1, WO 2008/105958 A2, WO 2017/090019 A2, WO 2016/120207 A1 and GB 2 490 721 A.

It is an object of the present disclosure to provide improvements relating to drug delivery devices and/or their operation.

This object is achieved by the subject-matter of the independent claims. Advantageous embodiments and refinements may be subject to dependent claims or are contained elsewhere in the disclosure. Also, the present disclosure may contain subject-matter which relates to improvements of drug delivery devices but is not claimed be it dependently or independently.

### Summary

The invention is defined in claims 1 and 14. Advantageous embodiments are subject of the dependent claims.

According to one aspect, the present disclosure relates to a drug delivery device. The drug delivery device may be configured to perform a drug delivery operation, in particular when actuated or initiated by a user. According to another aspect, the present disclosure relates to a system comprising the drug delivery device. According to yet another aspect, the present disclosure relates to a method. The method may be a method for evaluating or influencing a drug delivery device, in particular an operation of the drug delivery device such as a delivery operation. The device evaluated in the method is preferably the disclosed drug delivery device.

It should be understood that features which are disclosed in relation to different aspects or embodiments do also apply for other aspects or embodiments. For example, features which are disclosed for the drug delivery device do also apply for the system and the method and vice versa. Also, features disclosed in conjunction with different embodiments can be combined with one another.

In an embodiment, the drug delivery device comprises a thermal coupling surface. The thermal coupling surface may be arranged and/or configured to transfer, e.g. to receive and/or emit, thermal energy, e.g. heat. The thermal coupling surface may be configured to be coupled thermally to another element. The thermal coupling surface may be formed by a thermally conductive material, e.g. a metal or metal alloy. Regions laterally surrounding or adjacent to the thermal coupling surface are formed of a material having lower thermal conductivity.

Typically, plastics have lower thermal conductivity than metals. For the energy transfer, the thermal coupling surface may be brought into thermal conductive connection, e.g. into mechanical contact, with the surface of another element with which the energy transfer should take place, e.g. the skin of the subject which should receive the drug to be delivered with the device. The drug delivery device may be configured such that thermal energy , e.g. heat, is guided away from the thermal coupling surface after it has been received by the thermal coupling surface.

In an embodiment, the drug delivery device comprises a thermally controlled or operated member. The thermally controlled member may change its state in response to thermal energy provided to it. The thermally controlled member may be thermally conductively connected to the thermal coupling surface. The thermally controlled member may be configured to operate in response to the thermal energy transferred through the thermal coupling surface. Accordingly, thermal energy may be used within the drug delivery device such as to monitor an operation of the device, evaluate an operation of the device, control an operation of the device, and/or influence an operation of the device. The operation may be the drug delivery operation.

In an embodiment, the thermally controlled member is a thermal energy conversion member. The thermal energy conversion member may be arranged and/or configured to convert thermal energy into non-thermal energy, in particular thermal energy transferred through the thermal coupling surface, e.g. to the thermal energy conversion member. In the device the converted non-thermal energy may be used to evaluate, monitor and/or influence the operation of the drug delivery device.

In an embodiment, the thermal energy is supplied to the thermally controlled member through the thermal coupling surface. The thermal coupling surface may, therefore, act as an energy input surface.

In an embodiment, the non-thermal energy is electrical energy or mechanical energy, e.g. kinetic energy.

In an embodiment, the non-thermal energy is kinetic energy for moving or potential energy which is convertable into kinetic energy for moving one part of the drug delivery device, e.g. the thermal energy conversion member or a region thereof, relative to another part of the drug delivery device, particularly in response to thermal energy supplied to the thermal energy conversion member.

In an embodiment, the thermally controlled member is a movable member which may be moved in response to thermal energy provided to the thermally controlled member and/or leaving the member. Consequently, the thermal energy may be used to influence the operation of the drug delivery device.

In an embodiment, the thermally controlled member is an electrical or electronic member. The thermally controlled member may be operatively connected to an electrical power supply of the device and/or to other electrical or electronic components or elements of the device. The thermal energy provided to the member may cause a change of an electrical characteristic of a component and/or generation of an electrical signal in response to the thermal energy.

In an embodiment, the thermally controlled member is an electrical, preferably electronic, temperature sensor or an electrical, preferably electronic, temperature triggered or triggerable switch. Accordingly, the thermally controlled member may operate in response to a temperature at the thermal coupling surface. Thus, the operation of the drug delivery device may be controlled by the temperature at the thermal coupling surface. A switch for example, when triggered, may activate an electrical drive mechanism or an electronic control unit when a predetermined temperature is present at the thermal coupling surface. The temperature sensor may permit retrieving information, e.g. electronically, about the current temperature at the thermal coupling surface. The temperature sensor may be an active sensor which generates an electrical signal indicative for the temperature at the thermal coupling surface or a passive sensor which changes an electrical characteristic dependent on the temperature at thermal coupling surface, e.g. a sensor which changes its electrical resistance depending on the temperature at the coupling surface such as a thermistor, e.g. a PTC component or an NTC component.

In an embodiment, the thermally controlled member, e.g. a thermal energy conversion member, is configured to generate an electrical signal in response to thermal energy and/or a change in thermal energy. The thermal energy may be thermal energy supplied to the thermally controlled member via the thermal coupling surface.

In an embodiment, the electrical signal is indicative for the temperature of an element which is or is to be thermally conductively connected to, for example in mechanical contact with, the thermal coupling surface. Accordingly, the device may be configured to operate dependent on the temperature at the coupling surface and/or the temperature at the thermal coupling surface may be recorded, monitored and/or stored in the drug delivery device or in an external component or system such as in a computer system.

The thermally controlled member, e.g. a region thereof or the entire member, is arranged and/or configured to move in response to thermal energy transferred through the thermal coupling surface. The thermally controlled member may convert the thermal energy into kinetic energy. The movement is used for the operation of the drug delivery device, e.g. to trigger an electrical switch and/or to release a mechanical lock. The respective event, i.e. the release of the mechanical lock and/or the triggering of the switch, may render the device of operational, e.g. a delivery operation may only be initiated after the event. For example, releasing the mechanical lock may enable movement of two components or elements of the device relative to one another in a direction where the movement was not possible when the mechanical lock was established.

In an embodiment, the thermally controlled member is a bimetallic member. In a bimetallic member two metal elements of different metals may be used which are thermally conductively connected to each other and have different coefficients of thermal expansion. Once thermal energy is supplied to the bimetallic member, e.g. to raise the temperature of the member, the two metal elements will expand differently which may result in movement of at least a portion of the bimetallic member.

In an embodiment, the thermally controlled member is designed to operate at a temperature greater than or equal to one of the following values: 22 °C, 23 °C, 24 °C, 25 °C, 26 °C, 27 °C, 28 °C, 29 °C, 30 °C, 31 °C, 32 °C, 33 °C, 34 °C.

In an embodiment, the operating temperature is expediently greater than or equal to the ambient temperature.

In an embodiment, the thermally controlled member is designed to operate at a temperature less than or equal to one of the following values: 44 °C, 43 °C, 42 °C, 41 °C, 40 °C, 39 °C, 38 °C, 37 °C, 36 °C, 35 °C, 34 °C, 33 °C, 32° C, 31 °C, 30 °C, 29 °C.

Temperature ranges, e.g. operating temperature ranges, may be formed by combining upper and lower boundaries for the range from arbitrary values selected from the lists mentioned above such as, for example 28 to 36 °C. The values may be selected from different lists or from the same list.

It may be advantageous to make the operating temperature range narrow as possible, e.g. with a maximum of 10 °C, of 8 °C or of 5 °C of the difference between the lower and upper bound of the temperatue range, as, in this case, by choosing the temperature range appropriately it is very likely that the user's skin contacts the thermal coupling surface if an element with a temperature within that range contacts the thermal coupling surface . Also choosing the operating temperature range narrowly reduces the risk that the device is operated in the wrong way by contacting a wrong member which, by chance, has a temperature in the desired operating temperature range.

In an embodiment, the thermally controlled member is designed to operate at the regular skin temperature or temperatures or the regular body temperature or temperatures of a mammal, preferably a human. The operating temperature range expediently covers the regular skin temperature of a human and/or the regular body temperature of a human. The regular skin temperature of the human may be between 32 and 34 °C at regular ambient temperatures. At 15°C ambient temperature the skin temperature may be as low as 24 °C, for example. At higher ambient temperatures, the skin temperature may reach 36 °C. As opposed to the skin temperature, the body temperature is usually higher such as between 35 and 43 °C, e.g. about 37 °C.

It may advantageous if the drug in the medicament container is of a temperature close to the body temperature. This is more comfortable for the user than a delivery of too hot or cold drugs. In an embodiment, the skin temperature can be used as an indicator whether the drug delivery will be performed into the correct structure.

In an embodiment, the drug delivery device comprises a housing. The housing may house further (e.g. internal) components of the drug delivery device such as one or more members of a drive mechanism operable to dispense drug from the delivery device in the drug delivery operation.

In an embodiment, the drug delivery device comprises a receptacle. The receptacle may be provided in the housing or, e.g. permanently or releasably, connected to the housing. The receptacle may be designed to receive a medicament container, such as a cartridge or syringe. In the drug delivery device, the medicament container may be received in the receptacle or the receptacle may be container-free and configured to be equipped with the container.

In an embodiment, the drug delivery device comprises a drive mechanism. The drive mechanism may be operable to drive a drug delivery operation. The drive mechanism may comprise at least one movable drive member. The movable drive member may be moved with respect to the housing in order to deliver medicament from the container through a dispensing opening of the drug delivery device during the drug delivery operation. The movable drive member may be movable with respect to the housing and/or relative to the medicament container. The movable drive member may be a plunger, for example. The movable drive member may be biased, such as by a spring, to drive the dispensing operation when the bias is released.

In an embodiment, the thermal coupling surface is an exterior surface of the drug delivery device or an interior surface of the drug delivery device. An exterior surface of the device is accessible from the exterior during and/or before the drug delivery operation occurs. The exterior surface may be touched by the user. An interior surface, however, may be hidden during the drug delivery operation and not be configured to be touched by the user. To the contrary, mechanical contact connection to the interior surface may be prevented, e.g. by the housing.

In an embodiment, the thermal coupling surface is associated with the receptacle of the drug delivery device. The thermal coupling surface may be thermally conductively connected to the medicament container when the medicament container is arranged in the receptacle. Accordingly, via the thermal coupling surface information can be gathered on the temperature of the medicament container and, in consequence, of the drug contained in the container. For example, if the temperature of the medicament of drug and the container is too low or too high, operation of the drug delivery device may be prevented such as electronically, e.g. due to an electronic control unit not issuing the necessary command, or mechanically, e.g. by a mechanical lock which is not released as the thermal energy is insufficient.

In an embodiment, the drug delivery device is configured such that, in response to thermal energy supplied to the thermal coupling surface, a mechanical lock, e.g. in the interior of the drug delivery device, is released. Here, "in response to thermal energy supplied to the thermal coupling surface" may cover a conversion of the thermal energy into kinetic energy or electrical energy and a change of an electrical characteristic of an electrical thermally controlled member. The mechanical lock, when established, may prevent the movement of an element of the device, preferably an element which is required to be moved in order to initiate the drug delivery operation. The mechanical lock, when released, may allow movement of the element in the direction which was previously blocked. Thus, when the mechanical lock is released, a delivery operation may be triggered. The element may be a movable member which is preferably movable relative to a housing of the drug delivery device in order to initiate the drug delivery operation and/or before the drug delivery operation is initiated. The element may be a trigger member which is provided for initiation of a delivery operation. The trigger member may be a trigger button such as a button at the proximal end of the drug delivery device or a needle shroud, e.g. at the distal end of the drug delivery device. The thermal coupling surface may be provided on the movable member or on the housing, for example.

The drug delivery device comprises an activation surface. The activation surface is arranged to be contacted by the user to initiate a drug delivery operation of the drug delivery device. The activation surface may be a surface of the trigger member, e.g. a distal surface, i.e. a surface facing in the distal direction, or a proximal surface, i.e. a surface facing in the proximal direction. The thermal coupling surface is accessible on the activation surface e.g. arranged on the activation surface. Therefore, the thermal energy supplied via the thermal coupling surface may be supplied by the element mechanically contacted by the activation surface, preferably skin. If thermal energy is supplied to the thermal coupling surface on the activation surface, this energy can be used to determine whether an element contacting the thermal coupling surface has or had a desired temperature, for example. In an embodiment, the drug delivery device comprises a bearing surface. The bearing surface may be arranged to contact the skin of a user or patient, preferably during and/or before a drug delivery operation, e.g. when the delivery operation is initiated or only after the delivery operation has been initiated. The thermal coupling surface may be accessible on the bearing surface, e.g. arranged on the bearing surface. Therefore, the thermal energy supplied via the thermal coupling surface may be supplied by the element mechanically contacted by the bearing surface, preferably skin. If thermal energy is supplied to the thermal coupling surface on the bearing surface, this energy can be used to determine whether an element contacting the thermal coupling surface has or had a desired temperature. The bearing surface may be adjacent to a target region on the skin for the drug delivery operation, e.g. a region into which the medicament from the medicament container should be delivered, e.g. injected. Accordingly, the thermal energy may be supplied to the thermal coupling surface close to the target region. If the thermally controlled member is adjusted to the temperature of the target region, which it generally may be, the thermal energy is supplied close to the target region. In this way, a particularly reliable operation of the thermally controlled member may be achieved. The bearing surface may be different from the activation surface. The bearing surface may be the same surface as the activation surface. The bearing surface may be provided at the distal end of the drug delivery device and/or it may be a distal surface.

In an embodiment, a thermal coupling surface may be provided on the activation surface and a thermal coupling surface may be provided on the bearing surface.

In an embodiment, the activation surface is movable relative to the bearing surface. A thermal coupling surface on the bearing surface may be arranged and configured to contact the skin only after the activation surface has been moved from a first or initial position to a second or end position. In the end position the drug delivery operation has, preferably, already been initiated. Accordingly, when the thermal coupling surface on the bearing surface is contacted by the skin, it can be guaranteed that the drug delivery operation has been initiated. Before the bearing surface is contacted by the skin, the same region of the skin or an adjacent region of the skin may contact the activation surface and move the activation surface towards the bearing surface.

In an embodiment, the drug delivery device comprises an electronic control unit, such as a microcontroller or microprocessor. The thermally controlled member may be operatively coupled or coupleable to the electronic control unit. Accordingly, the thermally controlled member may communicate with the electronic control unit. Thus, the electronic control unit may operate based on information, signals or data supplied to it from the thermally controlled member. For example, data signals indicative for the temperature at the thermal coupling surface may be transmitted from the thermally controlled member to electronic control unit.

In an embodiment, the operation of the drive mechanism is controlled by the electronic control unit. For example, the electronic control unit may issue a command to a drive control member which in response to the command moves a movable member, e.g. motor driven, in order to drive the drug delivery operation.

In an embodiment, the drug delivery device is configured such that the electronic control unit permits initiation of the drug delivery operator only if a temperature at the thermal coupling surface is within a predetermined temperature range. For example, the control unit may activate the drive mechanism only if the temperature is within the predetermined temperature range and/or release a mechanical lock only if the temperature is within the predetermined temperature range. The predetermined temperature range may be chosen to be characteristic for the skin temperature or for the body temperature of a human.

In an embodiment, the drug delivery device is configured to store and/or transmit data on the temperature at the thermal coupling surface, in particular when a delivery operation has been initiated and/or while the delivery operation is being performed. The storage and/or the data transmission may be controlled by the electronic control unit. The data transmission may occur from the drug delivery device to an evaluation unit, which may be configured to evaluate the temperature data. In this way, it can be assessed, e.g. externally, whether the temperature at the thermal coupling surface was within a predetermined temperature range at initiation and/or during performance of the drug delivery operation. Accordingly, if the temperature was within a range characteristic for the skin temperature, e.g. 24 °C to 36 °C, of a human and the thermal coupling surface is arranged to contact the skin, it can be assumed that the drug delivery device was used to inject drug into a human body. If the thermal coupling surface is thermally conductively connected to the medicament container it can be judged whether the temperature of the drug is or was within the right temperature range.

In an embodiment, the system comprises the drug delivery device and an evaluation unit. The evaluation unit may be integrated into or separate from but operatively coupled or coupleable to the drug delivery device, e.g. using a wired or wireless connection. The drug delivery device may be configured to transfer temperature data which is indicative for the temperature at the thermal coupling surface, in particular during and/or at the initiation of the delivery operation, to the evaluation unit. The evaluation unit may be configured to evaluate the temperature data, e.g. compare it with a predetermined temperature or temperature range. Depending on the result of the evaluation different outputs may be generated by the evaluation unit, e.g. that a predetermined temperature range was met or not. Accordingly the user, patient or caregiver can take the appropriate measures depending on the result of the evaluation, e.g. on the output.

In an embodiment, the method comprises the step of comparing a temperature at the thermal coupling surface with a predetermined temperature or temperature range, e.g. before, during and/or after the delivery operation. The temperature may be derived from the thermally controlled member. The temperature may be the current temperature at the thermal coupling surface. If the temperature is within the predetermined temperature range, initiation of the delivery operation may be allowed. If it is not, initiation may be prevented. Alternatively or additionally, if the temperature is within the range, an according output may be transmitted to a further device such as a computer system which indicates that the temperature was correct. Alternatively or additionally, if the temperature is not within the range, an according output may be transmitted to further device indicating that the temperature was not correct. Depending on the outcome of the evaluation, appropriate measures may be taken.

In a particularly advantageous embodiment, a drug delivery device is provided, the drug delivery device comprising:
- a thermal coupling surface which is arranged and configured to transfer thermal energy,
- a thermally controlled member, which is thermally conductively connected to the thermal coupling surface, wherein the thermally controlled member is configured to operate in response to thermal energy transferred through the thermal coupling surface, and wherein the drug delivery device is configured to perform a drug delivery operation.

Further features, advantages and expediences of the present disclosure will become apparent from the following description of the exemplary embodiments in conjunction with the drawings.

### Brief description of the drawings

Figures 1A and 1B illustrate different schematic views of an embodiment of a drug delivery device.
Figures 2A and 2B illustrate an embodiment of a drug delivery device in two different states.
Figure 3 illustrates another embodiment of a drug delivery device on the basis of a schematic sectional view.
Figure 4 illustrates schematically electronic elements of an embodiment of a drug delivery device.

### Description of exemplary embodiments

In the drawings, identical elements, identically acting elements and elements of the same kind may be designated using the same reference numerals. It should be noted that features which are disclosed further above and/or further below can be combined with one another and/or are considered to be disclosed independently from each other, i.e. without an inextricable link to the other features disclosed in the same context.

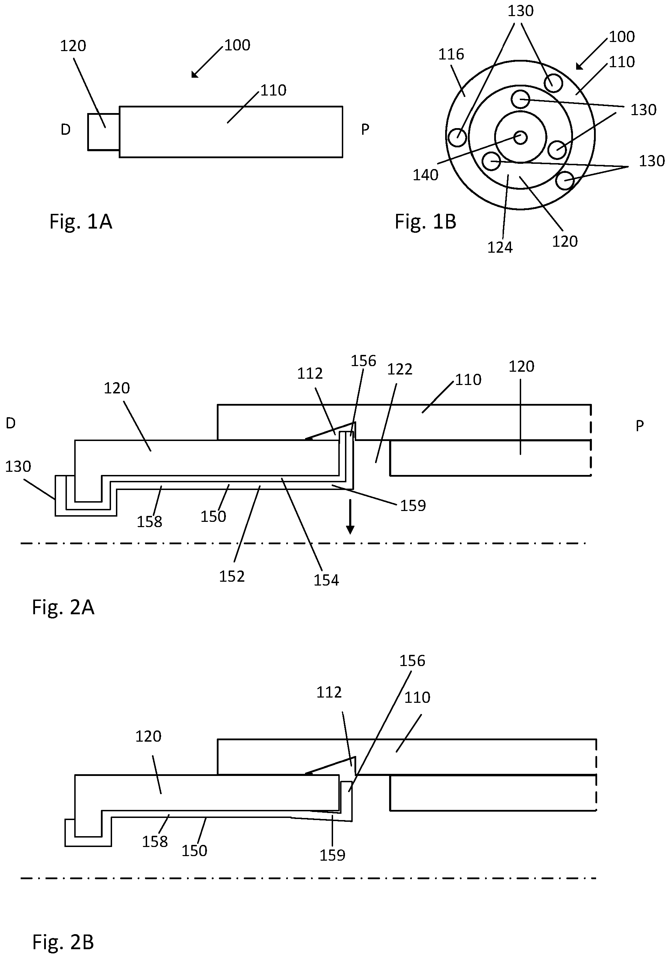

Figures 1A and 1B illustrate different schematic views of an embodiment of a drug delivery device 100. Figure 1A shows a schematic side view of the drug delivery device 100 and figure 1B shows a top view onto a distal end of the drug delivery device.

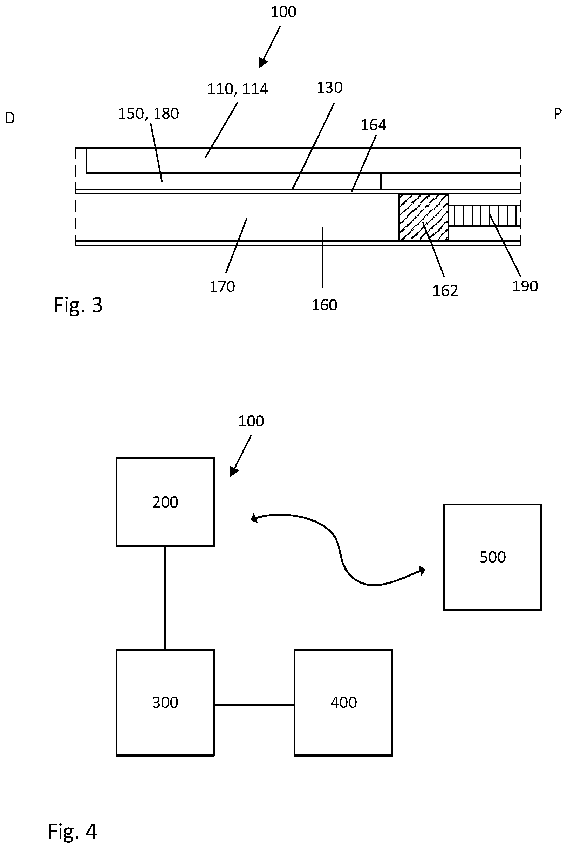

The drug delivery device 100 comprises a housing 110. Within the housing 110, a drive mechanism may be retained. The drug delivery device may be operable to drive a dispensing or delivery operation to dispense a drug, such as liquid drug, from a medicament container received or receivable in the drug delivery device and, especially in the housing 110. The housing 110 may be designed to receive a medicament container in the interior. Within the housing 110 a container receptacle may be provided which may receive and/or retain the container (not explicitly shown). The container receptacle may secure the medicament container against movement with respect to the housing or be movable together with the container relative to the housing. The container may be a cartridge or a syringe. The container may hold an amount of medicament sufficient for a plurality of doses to be delivered by the device in separate delivery operations or just a single dose of drug in one delivery operation. The drug delivery device may comprise a dose setting mechanism, i.e. a mechanism which permits to select the size of the dose to be dispensed, e.g. from a variety of possible sizes. The dose setting mechanism may comprise at least one dose setting member which is movably connected to the housing and operable to be moved relative to the housing to set a dose of drug (not explicitly shown, see drug 170 in figure 3). The drive mechanism may comprise a plunger (not explicitly shown, see plunger 190 in figure 3). For the delivery operation, the plunger may be driven into a distal direction relative to the housing 110.

In the context of the present disclosure "distal" or "distal direction" may mean a, preferably axial, direction towards a dispensing end of the device and/or away from an end of the device which is remote from, e.g. opposite to, the dispensing end. The "distal end" may mean an end of an element of the device which is or is to be arranged to face towards or be closest to the dispensing end of the device or face away or be remote from an end of the device which is remote from, e.g. opposite to, the dispensing end.

In the context of the present disclosure "proximal" or "proximal direction" may mean a, preferably axial, direction away from a dispensing end of the device and/or towards an end of the device which is remote from, e.g. opposite to, the dispensing end. The "proximal end" may mean an end of an element of the device which is or is to be arranged to face away or to be remote from the dispensing end of the device or face towards an end of the device which is remote from, e.g. opposite to, the dispensing end. In figure 1A, the proximal end of the device is designated with "P" and the distal end is designated with "D", where the dispensing end of the device is on the left where the distal end is situated.

The distal movement of the plunger may cause delivery of drug from the container via an outlet of the device, e.g. an end of a needle or another opening, facing in the distal direction. The plunger, during the delivery operation, may be driven by energy provided to the plunger by way of an energy storage member, where the energy is released during the delivery operation. The energy storage member may be a drive spring for example (not explicitly shown). However, pressurized gas may also be used to provide the energy for the delivery operation. The energy stored in the energy storage member may be pre-stored in the device. That is to say, the user does not need to provide the energy for the delivery operation rather the energy stored in the energy storage member during manufacturing. In other words, the depicted drug delivery device 100 may be an autoinjector.

A movable member 120 is movably coupled to the housing 110. The movable member 120 may be retained within housing 110 such that it cannot be removed. A portion of the movable member 120 may, at least in an initial or first position, protrude from a distal end of the housing 110 as depicted in figure 1A. The movable member 120 may be movable relative to the housing in the proximal direction from the first position into a second position, e.g. in order to trigger a delivery operation of the drive mechanism retained in the housing 110 and/or for a needle insertion step which inserts the needle into the skin of the user. For example, the movable member 120 may be moved further into the housing in order to trigger the delivery operation, i.e. it may act as a trigger member. Alternatively or additionally, the movable member 120 may be a needle cover or needle shroud which is movably coupled to the housing in order to prevent unintentional contact between the user and a needle in the interior of the device and/or to shield the needle from the user such that the needle is invisible for the user before, during and/or after completion of the drug delivery operation. The movable member 120 may cover the needle in the initial position depicted in figure 1A. If the movable member 120 is not used to trigger a delivery operation, a separate trigger member such as a trigger button, e.g. at the proximal end of the housing may be used for this purpose (not shown). The needle may be retained in the housing. For example, the needle may be, preferably permanently, attached to the medicament container which, in this case may be a syringe, e.g. with a staked needle. Alternatively, the needle may be a needle separate from the medicament container. In this case, the medicament container may be a cartridge, for example. It should be noted that the disclosed concepts do not only apply to autoinjectors. Rather, the disclosed concepts also apply to other drug delivery devices such as drug delivery devices for delivering user-settable doses or fixed doses with or without providing a force-assist during dose delivery. The concepts may apply to pen-type devices, such as pen-type injectors. The devices may be needle-based or needle-free. The devices may be carry-on items, e.g. sized and shaped to be stored in a bag. The devices may be suitable to be used by medically untrained people and/or for self-administration of drugs. The devices may be designed for subcutaneous injection procedures. It should also be noted, that the present disclosure is not restricted to drug delivery devices with the movable member 120 at the distal end. Rather, also other types of devices, e.g. with rigid distal ends, may be used in the present context.

The distal end of the drug delivery device 100 may be provided with a removable cap which can be detached before a delivery operation is conducted. In the situation depicted in figures 1A and 1B, the cap (if present) has been removed, if applicable together with a soft or rigid needle shield which covers the needle distally. In figure 1B the distal end of a needle 140 can be seen with the delivery opening via which drug may leave the drug delivery device. The member 120 and/or the housing 110 may surround the needle 140 when seen in top view and/or also axially. Thus, laterally, the needle may be shielded by way of the movable member 120 which acts as needle shield or needle shroud.

Figure 1B illustrates that one or a plurality of thermal coupling surfaces or surface regions 130 may be accessible on the distal end side of the drug delivery device. The respective surface may be a distal surface. That is to say the respective thermal coupling surface may be arranged to thermally and/or mechanically contact the skin of a body into which the drug delivery device should deliver drug during the delivery operation. In the depicted arrangement a plurality of coupling surfaces 130 are provided on each, a distal end of the housing 110 and on a distal end of the movable member 120. However, it should be noted, that not both of the housing and the movable member need to contain thermal coupling surfaces. It is sufficient that at least one thermal coupling surface is accessible at the distal end which could be on either one of these members, for example. Also, if no movable member is provided at the distal end of the device or even with the movable member at the distal end, a thermal coupling surface may be provided on a distal surface of the housing 110 only. The respective thermal coupling surface 130 may be provided by a thermally conductive material, e.g. a metal or a metal alloy. A region surrounding the thermal coupling surface 130 or arranged laterally adjacent to the thermal couplings surface, e.g. when seen in top view, may be of a material with a lower thermal conductivity than the thermal coupling surface, e.g. plastic. The respective thermal coupling surface 130 may be a recessed region, a protruding region or a region flush with the adjacent region. In the depicted configuration the thermal coupling surfaces 130 are circumferentially disposed on the element on which they are provided, here the housing 110 or the movable member 120. Thermal coupling surfaces 130 on different elements may be angularly offset from one another.

The respective thermal coupling surface 130 may be arranged and configured to receive thermal energy from an element on which the respective coupling surface bears, e.g. the user's skin, when the device is operated. Thus, the respective surface may be accessible on a bearing surface of the device, e.g. a distal surface of the movable member 120 and/or the housing 110. If one or more thermal coupling surfaces are provided on a distal surface of the device, the respective surface can be used for receiving thermal energy from the user close to the region where the delivery operation into the user's body is effected, such as close to dispensing opening or the needle, e.g. laterally adjoining the needle.

If at least one thermal coupling surface 130 is provided on each of two members which are movable relative to one another - housing 110 and movable member 120 in the depicted embodiment - the thermal conductive connection between the thermal coupling surface on the two members and the target surface, e.g. the skin, may occur during different stages of operation of the device, e.g. during initation or triggering of the drug delivery operation and after the delivery operation has been initiated. For example, initially, the movable member 120 and a thermal coupling surface 130 on this member may be thermally and/or mechanically contacted by the skin and, after the movable member has been moved relative to the housing, the thermal coupling surface 130 on the housing 110 may be thermally and/or mechanically contacted by the skin. This arrangement facilitates distinguishing between different stages of operation via thermal energy provided to the respective thermal coupling surface 130 from the exterior. It is, however, also possible to have a thermal coupling surface 130 only on the movable member 120 or only on the housing 110. If a thermal coupling surface 130 is provided on the movable member the initial skin contact can be used to transfer thermal energy to the thermally controlled member, e.g. to release a mechanical lock as will be further discussed below. If a thermal coupling surface is provided on the housing 110, the thermal energy may be transferred from the skin to that coupling surface when the housing has assumed its final position relative to the skin, e.g. after the delivery operation has been initiated such as by the movable member when it has reached the second position. Accordingly, in this case the thermal energy may be used to provide an indication that the delivery operation has been triggered and/or information on the temperature of the surface into which the medicament or drug has been delivered as will be discussed further below. The distal surface 116 of the housing 110 may be a bearing surface which is contacted by the skin before, during and/or after completiion of the drug delivery operation. The distal surface 124 of the movable member may be an activation surface - if the movable member is used to initiate the dose delivery operation - and/or a bearing surface.

The thermal energy which is provided to the respective thermal coupling surface may be transferred to a thermally controlled member. The thermally controlled member may be a thermal energy conversion member. Thermal energy transferred to the thermal energy conversion member may be converted into non-thermal energy by the thermal energy conversion member, such as into mechanical potential and/or kinetic energy in order to move elements of the device relative to one another and/or electrical energy such as to generate an electrical signal which can be used for controlling operation of the device by an electronic control unit which is described later on in more detail. Alternatively or additionally to energy conversion, thermal energy supplied to the thermally controlled member may change a characteristic, e.g. an electrical characteristic, of the thermally controlled member. The change in the characteristic may be used to influence and/or evaluate the operation of the device.

The respective thermal coupling surface 130 may be thermally conductively connected to a thermally controlled member. The thermally controlled member may be coupled to two different thermal coupling surfaces, when a plurality of such surfaces is provided, or two different thermal coupling surfaces may be thermally separated from one another and thermally conductively connected to two different thermally controlled members. If thermal coupling surfaces are provided in two different members which are movable relative to one another it is expedient that these coupling surfaces are thermally conductively connected to different thermally controlled members. In other words, the device may comprise one or a plurality of thermally controlled members.

Examples of thermally controlled members include bimetallic members, e.g. bimetallic strips, which are configured to convert thermal energy into mechanical energy, e.g. kinetic energy. The thermally controlled member may be a temperature sensor, e.g. an active or passive sensor, or temperature controlled switch. Active temperature sensors as thermally controlled members may be configured to convert thermal energy into electrical energy, such as electrical sensor signals, which can be communicated to an electronic control unit which may be provided to control operation of an, e.g. electrically and/or motor driven, drive mechanism of the drug delivery device. Active temperature sensors may generate an electrical signal indicative for the temperature. Such active sensors may be semiconductor-based sensors, e.g. comprising circuitry. Passive electrical temperature sensors may change an electrical characteristic, e.g. the resistance or resistivity, dependent of the temperature. Examples for passive temperature sensors which have a temperature dependent resistance include PTC thermistors, or NTC thermistors (PTC: positive temperature coefficient; NTC: negative temperature coefficient). The temperature sensed by the respective sensor may be determined by or equal to the temperature at the thermal coupling surface. Instead of sensors which provide information on the specific temperature, temperature triggered switches may be used which are triggered when the temperature is above or below a predetermined threshold value.

Figures 2A and 2B illustrate an embodiment of a drug delivery device. For example, the device may be a device as discussed further above in conjunction with figures 1A and 1B. Only the upper half is shown as indicated by the dashed line.

Figure 2A illustrates a sectional view of a part of the distal region of the drug delivery device of figure 1A and 1B. Specifically, the movable member 120 and the housing 110 are depicted. Also, a thermally controlled member 150 is depicted which is thermally conductively connected to the thermal coupling surface 130 provided at the distal end of the device 100. In the depicted embodiment, the thermal coupling surface 130 is a surface of the thermally controlled member 150 which is accessible on the distal end. However, it should be readily understood that, in particular when a sensor is used as a thermally controlled member but not restricted to that configuration, the thermal coupling surface 130 could also be a surface separate from the thermally controlled member but only thermally conductively connected to that member. The thermally controlled member 150 extends from the distal surface of the movable member 120 into the interior of the, e.g. sleeve-like, movable member, along the an inner surface of the movable member 120, preferably in the axial direction.

In the depicted embodiment, the thermally controlled member 150 is a bimetallic member or bimetallic strip. The thermally controlled member 150 comprises a first metal or metal alloy layer 152 which is connected to a second metal or metal alloy layer 154. The materials of layers 152 and 154 do have different thermal expansion coefficients and/or the layers 152 and 154 are thermally conductively connected, e.g via welding, a force-fit connection or a form-fit connection. Accordingly, when thermal energy is supplied to one layer, this energy is also transferred to the second layer, especially when considering the high thermal conductivity of metals.

When thermal energy is supplied to the thermal coupling surface and the temperature of the member 150 is raised the different expansion coefficients result in different expansion behaviors of the layers 152 and 154 which causes movement of at least a portion of the thermally controlled member. The thermally controlled member 150 may comprise a fixed portion 158, which is preferably axially and rotationally fixed to another element, e.g. the movable member 120, and a movable portion 159 which is movable relative to the fixed portion, e.g. pivotable, relative to the fixed portion. The fixed portion may secure the thermally controlled member to the movable member. The thermally induced movement of the thermally controlled member may be used to unlock a mechanical lock which prevents movement of the movable member 120, e.g. in the proximal direction, such as movement required for triggering the drive mechanism and/or for piercing the needle into the skin. In the depicted embodiment the mechanical lock is provided by a locking portion 156 of the thermally controlled member 150, which is arranged to engage a mechanical stop 112 provided on the housing 110 in the situation depicted in figure 1A. The locking portion 156 may be proximally offset from the thermal coupling surface 130 and/or from the fixed portion 158. The locking portion may be situsated in the end reion of the movable portion 159. It should be noted, that the mechanical lock could also be implemented differently such as by a movable element which is moved under control of an electronic control unit where the movement depends on the temperature at the thermal coupling surface. Also, the movement of the thermally controlled member, e.g. a bimetallic strip could be used to trigger an electric switch which causes the electronic control unit to remove a mechanical lock. However, if the thermally controlled member 150 is used to establish the lock, the mechanism is particularly easy to implement and cost-effective. The mechanical stop 112 in the housing is provided by a recess in the depicted embodiment. However, a protrusion could also be used. Moreover, the mechanical stop does not need to be implemented within the housing but just needs to prevent, e.g. proximal, movement relative to the housing in order to prevent the a movement of the movable member 120 from the first position to the second position required for the operation of the device 100. The thermally controlled member 150, particularly its locking portion may protrude from an interior of the movable member 120 through an opening 122, e.g. a radial opening, in the movable member towards the exterior of the movable member. On the outside of the movable member 120, e.g. on an interior surface of the housing 110, the mechanical stop 112 may be arranged and be engaged by the portion 156 of the thermally controlled member 150 when the mechanical lock is established.

By choosing the metals or metal alloys for the bimetallic member appropriately, it can be ensured that the release of the mechanical lock occurs when skin of a particular temperature such as the skin temperature of a human mechanically contacts the thermal coupling surface 130, e.g. skin of a temperature of about 32 to 34 °C or other temperatures possible for the human skin, and the metals or metal alloys are heated from the ambient temperature to the skin temperature. One of the layers 152 and 154, e.g. layer 152, may be made of or may comprise MnNiCu and the other one of the layers, e.g. layer 154, may be made of or comprise FeNi. However, other material pairs having different coefficients of thermal expansion may be suitable as well. The one layer of the layers 152 and 154 having the higher coefficient of thermal expansion is often also designated as the active layer, as its expansion/contraction predominantly drives the movement of the movable portion, and the layer with the lower coefficient of thermal expansion is often also designated as the passive layer as it follows the movement of the other layer. In the arrangement depicted in Figure 2A, layer 154, a portion of which is radially outwardly disposed relative to the adjacent portion of layer 152, may be the active layer and layer 152 may be the passive layer. This arrangement favours or induces a movement of the locking portion 156 and/or the movable portion 159 in the radial inward direction, e.g. when the thermally controlled member is heated from ambient temperature to skin temperature.

The locking portion 156 may be moved by 1 mm or more, 2 mm or more, or 3 mm or more relative to the housing, particularly radially, when releasing the mechanical lock, e.g. by 5 mm.

When the thermal energy is supplied to the thermal coupling surface 130 and the temperature in member 150 is raised, this causes movement of the locking portion 156 of the movable member in the direction of the arrow in figure 2A, i.e. radial inwards. The movable portion 159 is moved as well. As a result of this movement, the mechanical lock is released as depicted in figure 2B (here, the individual layers of the thermally controlled member 150 are no longer shown for simplicity reasons). As the lock has been released by the thermal energy provided to the coupling surface, movement of the movable member 120 in the proximal direction is now allowed.

Providing a thermally controlled release of an activation or lock mechanism for permitting initation of a (delivery) operation of a drug delivery device has the advantage that unintentional activation, i.e. activation when the device has not been placed in the desired region, especially on the skin of the patient, can be avoided or the risk can be at least reduced significantly.

It should be noted that the embodiment discussed above, i.e. releasing the mechanical lock depending on thermal input to the thermal coupling surface 130, could also be realized with an electronic temperature sensor as thermally controlled member 150. Then, for example based on an output signal of the sensor which is indicative for the temperature at the thermal coupling surface, a mechanical lock could be released, e.g. by the electronic control unit issuing an according command.

Having the thermal coupling surface at the activation and/or bearing surface with which the device contacts the skin of the user during use has the advantage that it can be determined whether the device during operation has contacted a surface with a temperature in a predetermined temperature range. The temperature range may be chosen such that it includes or consists of typical skin temperatures. For example, the temperature range may be the range between 29 and 35 °C or cover other temperatures customary for the skin. If the temperature determined before, during and/or after the delivery operation at the activation and/or bearing surface (for example the distal surfaces of the movable member 120 and/or the housing 110 shown in figures 1A and 1B) is within the predetermined range it is assumed that the delivery operation has occurred to deliver drug into a user's body. If the temperature is outside of that range, it can be assumed that an application of the drug into a body has not occurred. In the latter case, appropriate measures can be taken such as performing another dose delivery operation, if applicable under control of a different person than the first time, issuing an alarm, informing other people, etc.. Of course, this functionality is preferably implemented within a drug delivery device which comprises an electronic control unit such as discussed in conjunction with the figure 4 embodiment.

Figure 3 illustrates another embodiment of a drug delivery device on the basis of a schematic sectional view. The device may be one of the devices discussed previously. Figure 3 shows an interior of the housing 110 of the drug delivery device or of a container receptacle 114 within the interior of the housing. Also, figure 3 shows a portion of a medicament container 160, which contains an amount of liquid drug 170 corresponding to a single dose or a plurality of doses to be delivered by the drug delivery device 100. The container, for example a syringe or a cartridge, may comprise a movable stopper 162 which sealingly closes an interior of the container in the proximal direction. When the stopper 162 is moved distally within the interior of the container, drug will be expelled through the distal dispensing end of the device. Figure 3 also shows a plunger 190 of the drive mechanism of the drug delivery device. The plunger is arranged to move the stopper 162 distally during the drug delivery operation, thereby expelling drug 170 from the container 160, preferably under the bias of a drive spring (not shown).

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An examples of an oligonucleotide is, for example: mipomersen sodium (Kynamro^{®}), a cholesterol-reducing antisense therapeutic for the treatment of familial hypercholesterolemia.

Examples of DPP4 inhibitors are Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g.,

Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope of the present invention, which encompass such modifications and any and all equivalents thereof, provided they fall within the scope of the appended set of claims.

In the depicted embodiment, the thermal coupling surface 130 is thermally and/or mechanically connected to an external surface of the medicament container 160, e.g. formed by a wall 164 of the container, such that a temperature at the coupling surface is indicative for the temperature of the drug 170 within the container 160. The wall of the container is expediently thermally conductive. The surface 130 may contact the exterior surface of the wall of the container 160. The coupling surface 130 may be formed by the thermally controlled member 150, e.g., again, a bimetallic member or a temperature sensor, or by a thermal conductor 180, e.g. a metal strip, which is thermally conductively connected to an electronic temperature sensor or temperature controlled switch. The sensor may be configured to provide information on the temperature at the thermal coupling surface 130 and, consequently, on the temperature of the drug with in the container.

In case the temperature of the drug is not within a desired range, e.g. too hot or cold such that the user may feel uncomfortable when the drug would be delivered into the body, a delivery operation may be prevented in a similar manner as discussed above in conjunction with figures 2A and 2B, e.g. mechanically or electronically. The temperature range which is best accepted by the user may be close to the usual body temperature of a human, e.g. between 35 °C and 40 °C. Alternatively or additionally, the temperature of the drug could be logged by the electronic control unit into an electronic memory, which may be provided in the drug delivery device, during a storage term of the device such that before the device is operated, it may be determined whether the drug temperature has been kept within a predefined window or region during storage. In this way, it can be ascertained that temperature has not had negative effects on the drug 170 before the device is used.

Figure 4 illustrates schematically electronic elements of an embodiment of a drug delivery device. The depicted elements or a part thereof may be present in case the devices described further above implement electronics, which may or may not be the case.

The drug delivery device 100 comprises a temperature sensor 200 and, if appropriate, a thermal conductor circuit. The temperature sensor generates an electric sensor signal in response to the thermal energy supplied to the sensor via the thermal coupling surface 130 or changes its charactristics based on the temperature at the coupling surface. The temperature sensor 200 is operatively, preferably electrically conductively, connected, to an electronic control unit 300. The electronic control unit 300 may be or may comprise a microcontroller, an integrated circuit, such as an ASIC (application specific integrated circuit) or other digital circuitry or control elements. The unit may comprise a memory or storage such as to store software which governs the operation of the drug delivery device and/or to store data relating to the operation of the drug delivery device, e.g. temperature data. Alternatively or additionally to the sensor 200 the device may comprises a temperature triggered switch, e.g. implementing a bimetallic member to selectively close an electrical circuit. The electronic control unit further expediently comprises an electrical power supply such as a battery which is not explicitly shown. The electronic control unit 300 may be configured to control operation of the device, preferably based on input received from the temperature sensor 200.

The drug delivery device 100 may further comprise an electrical drive control member 400 such as a motor, for example. The drive control member 400 may operate the drive mechanism under control of the electronic control unit, e.g. advance the plunger via the motor. If the temperature indicative input fed to the electronic control unit by means of the sensor 200 is not within a predetermined range - e.g. the range characterisitc for the human skin or for a temperature of the medicament acceptable for delivery -, the electronic control unit may prevent operation of the drive control member, e.g. by not issuing a control command to the drive control member which would cause initiation of the delivery operation. If, however, the temperature is within the predetermined range, the control unit 300 may initiate the delivery operation. According considerations apply for a mechanical lock being released, e.g. by the drive control member.

Figure 4 further shows an evaluation unit, preferably an electronic evaluation unit 500. The evaluation unit 500 may be a unit separate from the drug delivery device or integrated into the device. In the former case, it is preferred, if the evaluation unit is connected or connectable to the drug delivery device via a wired or a wireless interface, e.g. NFC, Bluetooth or Wi-Fi. This is symbolized by the double arrow in figure 4. If the unit is integrated into the device, it may be integrated into the electronic control unit 300 or operatively coupled to the control unit. The evaluation unit may receive temperature data indicative for the temperature at the thermal coupling surface during the device operation and/or its initiation. Based on this temperature data, it can be decided whether the delivery operation has been made using drug of an appropriate temperature and/or into a structure having a surface having an appropriate temperature such as for the skin of the user. The information whether the drug and/or the surface into which the drug has been delivered has a temperature within a desired range and/or the information on the temperature at the thermal coupling surface may be communicated to a receiving device such as a mobile or non-mobile computing device of the user, a caregiver, parents of the user or other medically trained or untrained people interested in this information. The communication may be performed via a wired or a wireless interface, e.g. NFC, Bluetooth or Wi-Fi. The information can be used to assess whether the device has been used and/or used properly.

In summary, the present disclosure addresses various concepts to use thermal energy in the context of drug delivery devices, which is advantageous for various reasons as has been discussed above.

We note that features which are disclosed only in the introductory section should be considered as also being disclosed in the section relating to the exemplary embodiments and vice versa.

The scope of protection is not limited to the examples given herein above. Any invention disclosed herein is embodied in each novel characteristic and each combination of characteristics, which particularly includes every combination of any features which are stated in the claims, even if this feature or this combination of features is not explicitly stated in the claims or in the examples.

### Reference numerals

- 100: drug delivery device
- 110: housing
- 112: mechanical stop
- 114: receptacle
- 116: surface
- 120: movable member
- 122: opening
- 124: surface
- 130: thermal coupling surface
- 140: needle
- 150: thermally controlled member
- 152: metal layer
- 154: metal layer
- 156: locking portion
- 158: fixed portion
- 159: movable portion
- 160: container
- 162: stopper
- 164: wall
- 170: drug
- 180: thermal conductor
- 190: plunger
- 200: temperature sensor
- 300: electronic control unit
- 400: electrical drive control member
- 500: evaluation unit

## Claims

1. A drug delivery device (100), the drug delivery device comprising:
- a thermal coupling surface (130) which is arranged and configured to transfer thermal energy,
- a thermally controlled member (150), which is thermally conductively connected to the thermal coupling surface, wherein the thermally controlled member (150) is configured to operate in response to thermal energy transferred through the thermal coupling surface (130), and wherein the drug delivery device (100) is configured to perform a drug delivery operation, wherein the thermally controlled member (150) is arranged and configured to move in response to thermal energy transferred through the thermal coupling surface (130), wherein the movement is used for the operation of the drug delivery device;
wherein the drug delivery device (100) comprises an activation surface (124), wherein the activation surface is arranged to be contacted by a user to initiate the drug delivery operation of the drug delivery device, and wherein the thermal coupling surface (130) is accessible on the activation surface;
and
wherein regions laterally surrounding or adjacent to the thermal coupling surface are formed of a material having lower thermal conductivity than the thermal coupling surface.

2. The drug delivery device of claim 1
wherein the thermally controlled member (150) is an electrical, preferably electronic, temperature sensor or an electrical, preferably electronic, temperature triggerable switch.

3. The drug delivery device of any one of the preceding claims,
wherein the thermally controlled member (150) is a bimetallic member.

4. The drug delivery device of any one of the preceding claims,
wherein the thermally controlled member (150) is designed to operate at a temperature greater than or equal to one of the following values: 22 °C, 23 °C, 24 °C, 25 °C, 26 °C, 27 °C, 28 °C, 29 °C, 30 °C, 31 °C, 32 °C, 33 °C, 34 °C.

5. The drug delivery device of any one of the preceding claims,
wherein the thermally controlled member (150) is designed to operate at a temperature less than or equal to one of the following values: 44 °C, 43 °C, 42 °C, 41 °C, 40 °C, 39 °C, 38 °C, 37 °C, 36 °C, 35 °C, 34 °C, 33 °C, 32 °C, 31 °C, 30 °C, 29 °C.

6. The drug delivery device of any one of the preceding claims,
wherein the drug delivery device (100) comprises a housing (110) and a receptacle (114), which is provided in the housing or permanently or releasably connected to the housing, wherein the receptacle is designed to receive a medicament container (160) or wherein the medicament container is received in the receptacle, and wherein the drug delivery device comprises a drive mechanism, which is operable to drive the drug delivery operation, wherein the drive mechanism comprises at least one moveable drive member (190) which is configured to be moved with respect to the housing in order to deliver medicament from the medicament container through a dispensing opening of the drug delivery device during the drug delivery operation.

7. The drug delivery device of claim 6,
wherein the thermal coupling surface (130) is associated with the receptacle of the drug delivery device (100) such that the thermal coupling surface is thermally conductively connected to the medicament container, when the medicament container is arranged in the receptacle.

8. The drug delivery device of any one of the preceding claims,
wherein the drug delivery device (100) is configured such that in response to thermal energy supplied to the thermal coupling surface (130) a mechanical lock is released, wherein the mechanical lock, when established, prevents movement of an element of the device and, when the mechanical lock is released, the movement of the element is allowed.

9. The drug delivery device of claim 8, wherein the element is a trigger member for initiation of the drug delivery operation, and wherein the trigger member is a trigger button or a needle shroud.

10. The drug delivery device of any one of the preceding claims,
wherein the drug delivery device comprises a bearing surface (116), which is arranged to contact the skin of a user or patient during and/or before the drug delivery operation, wherein the thermal coupling surface (130) is accessible on the bearing surface.

11. The drug delivery device of any one of the preceding claims,
wherein the drug delivery device (100) comprises an electronic control unit (200), wherein the thermally controlled member (150) is operatively coupled or coupleable to the electronic control unit and wherein the drug delivery device is configured such that the electronic control unit permits initiation of the drug delivery operation only if a temperature at the thermal coupling surface (130) is within a predetermined temperature range.

12. The drug delivery device of any one of the preceding claims,
wherein the drug delivery device (100) is configured to store and/or transmit data on the temperature at the thermal coupling surface, in particular when the drug delivery operation has been initiated and/or while the drug delivery operation is being performed.

13. A system comprising the drug delivery device (100) of any one of the preceding claims and an evaluation unit (500) which is integrated into or operatively coupleable to the drug delivery device, wherein the drug delivery device is configured to transfer temperature data which is indicative for the temperature at the thermal coupling surface, in particular during a delivery operation, to the evaluation unit.

14. A method for evaluating a drug delivery device (100) according to any one of the preceding claims 1 to 12, comprising the step of comparing a temperature at the thermal coupling surface (130) with a predetermined temperature or temperature range, the temperature being preferably derived from the thermally controlled member, e.g. before, during or after the delivery operation.

## Patentansprüche

1. Medikamenten-Verabreichungsvorrichtung (100), wobei die Medikamenten-Verabreichungsvorrichtung umfasst:
- eine Wärmekopplungsfläche (130), die zum Übertragen von Wärmeenergie angeordnet und gestaltet ist,
- ein wärmegesteuertes Element (150), das wärmeleitend mit der Wärmekopplungsfläche verbunden ist, wobei das wärmegesteuerte Element (150) dafür gestaltet ist, als Reaktion auf durch die Wärmekopplungsfläche (130) übertragene Wärmeenergie zu arbeiten, und wobei die Medikamenten-Verabreichungsvorrichtung (100) dafür gestaltet ist, einen Medikamenten-Verabreichungsvorgang durchzuführen, wobei das wärmegesteuerte Element (150) dafür angeordnet und gestaltet ist, sich als Reaktion auf durch die Wärmekopplungsfläche (130) übertragene Wärmeenergie zu bewegen, wobei die Bewegung für den Betrieb der Medikamenten-Verabreichungsvorrichtung verwendet wird;
wobei die Medikamenten-Verabreichungsvorrichtung (100) eine Aktivierungsfläche (124) umfasst, wobei die Aktivierungsfläche zum Kontakt durch einen Benutzer angeordnet ist, um den Medikamenten-Verabreichungsvorgang der Medikamenten-Verabreichungsvorrichtung auszulösen, und wobei die Wärmekopplungsfläche (130) an der Aktivierungsfläche zugänglich ist;
und
wobei Bereiche, die die Wärmekopplungsfläche lateral umgeben oder an diese angrenzen, aus einem Material gebildet sind, das eine niedrigere Wärmeleitfähigkeit als die Wärmekopplungsfläche aufweist.

2. Medikamenten-Verabreichungsvorrichtung nach Anspruch 1, wobei das wärmegesteuerte Element (150) ein elektrischer, vorzugsweise elektronischer, Temperatursensor oder ein elektrischer, vorzugsweise elektronischer, temperaturauslösbarer Schalter ist.

3. Medikamenten-Verabreichungsvorrichtung nach einem der vorstehenden Ansprüche,
wobei das wärmegesteuerte Element (150) ein Bimetallelement ist.

4. Medikamenten-Verabreichungsvorrichtung nach einem der vorstehenden Ansprüche,
wobei das wärmegesteuerte Element (150) dafür ausgelegt ist, bei einer Temperatur von höher als oder gleich einem der folgenden Werte zu arbeiten: 22 °C, 23 °C, 24 °C, 25 °C, 26 °C, 27 °C, 28 °C, 29 °C, 30 °C, 31 °C, 32 °C, 33 °C, 34 °C.

5. Medikamenten-Verabreichungsvorrichtung nach einem der vorstehenden Ansprüche,
wobei das wärmegesteuerte Element (150) dafür ausgelegt ist, bei einer Temperatur von niedriger als oder gleich einem der folgenden Werte zu arbeiten: 44 °C, 43 °C, 42 °C, 41 °C, 40 °C, 39 °C, 38 °C, 37 °C, 36 °C, 35 °C, 34 °C, 33 °C, 32 °C, 31 °C, 30 °C, 29 °C.

6. Medikamenten-Verabreichungsvorrichtung nach einem der vorstehenden Ansprüche,
wobei die Medikamenten-Verabreichungsvorrichtung (100) ein Gehäuse (110) und eine Aufnahme (114), die in dem Gehäuse bereitgestellt oder dauerhaft oder lösbar mit dem Gehäuse verbunden ist, umfasst, wobei die Aufnahme dafür entworfen ist, einen Medikamentenbehälter (160) aufzunehmen, oder wobei der Medikamentenbehälter in der Aufnahme aufgenommen ist, und wobei die Medikamenten-Verabreichungsvorrichtung einen Antriebsmechanismus umfasst, der betreibbar ist, den Medikamenten-Verabreichungsvorgang anzutreiben, wobei der Antriebsmechanismus wenigstens ein bewegliches Antriebselement (190) umfasst, das dafür gestaltet ist, bezogen auf das Gehäuse bewegt zu werden, um bei dem Medikamenten-Verabreichungsvorgang Medikament aus dem Medikamentenbehälter durch eine Abgabeöffnung der Medikamenten-Verabreichungsvorrichtung zu verabreichen.

7. Medikamenten-Verabreichungsvorrichtung nach Anspruch 6, wobei die Wärmekopplungsfläche (130) mit der Aufnahme der Medikamenten-Verabreichungsvorrichtung (100) verbunden ist, so dass die Wärmekopplungsfläche wärmeleitend mit dem Medikamentenbehälter verbunden ist, wenn der Medikamentenbehälter in dem Behälter angeordnet ist.

8. Medikamenten-Verabreichungsvorrichtung nach einem der vorstehenden Ansprüche,
wobei die Medikamenten-Verabreichungsvorrichtung (100) so gestaltet ist, dass als Reaktion auf Wärmeenergie, die der Wärmekopplungsfläche (130) zugeführt wird, eine mechanische Verriegelung gelöst wird, wobei die mechanische Verriegelung, wenn eingerichtet, Bewegung eines Elements der Vorrichtung verhindert und, wenn die mechanische Verriegelung gelöst wird, die Bewegung des Elements erlaubt wird.

9. Medikamenten-Verabreichungsvorrichtung nach Anspruch 8, wobei das Element ein Auslöseelement zum Initiieren des Medikamenten-Verabreichungsvorgangs ist und wobei das Auslöseelement ein Auslöseknopf oder eine Nadelhülle ist.

10. Medikamenten-Verabreichungsvorrichtung nach einem der vorstehenden Ansprüche,
wobei die Medikamenten-Verabreichungsvorrichtung eine Anlagefläche (116) umfasst, die zum Kontakt mit der Haut eines Benutzers oder Patienten während und/oder vor dem Medikamenten-Verabreichungsvorgang angeordnet ist, wobei die Wärmekopplungsfläche (130) an der Anlagefläche zugänglich ist.

11. Medikamenten-Verabreichungsvorrichtung nach einem der vorstehenden Ansprüche,
wobei die Medikamenten-Verabreichungsvorrichtung (100) eine elektronische Steuereinheit (200) umfasst, wobei das wärmegesteuerte Element (150) mit der elektronischen Steuereinheit wirkgekoppelt oder -koppelbar ist und wobei die Medikamenten-Verabreichungsvorrichtung so gestaltet ist, dass die elektronische Steuereinheit Initiieren des Medikamenten-Verabreichungsvorgangs nur dann erlaubt, wenn eine Temperatur an der Wärmekopplungsfläche (130) innerhalb eines vorgegebenen Temperaturbereichs liegt.

12. Medikamenten-Verabreichungsvorrichtung nach einem der vorstehenden Ansprüche,
wobei die Medikamenten-Verabreichungsvorrichtung (100) dafür gestaltet ist, Daten über die Temperatur an der Wärmekopplungsfläche zu speichern und/oder zu übertragen, insbesondere wenn der Medikamenten-Verabreichungsvorgang ausgelöst worden ist und/oder während der Medikamenten-Verabreichungsvorgang durchgeführt wird.

13. System, umfassend die Medikamenten-Verabreichungsvorrichtung (100) nach einem der vorstehenden Ansprüche und eine Auswertungseinheit (500), die in die Medikamenten-Verabreichungsvorrichtung integriert ist oder damit wirkgekoppelt werden kann, wobei die Medikamenten-Verabreichungsvorrichtung dafür gestaltet ist, Temperaturdaten, die für die Temperatur an der Wärmekopplungsfläche indikativ sind, insbesondere während eines Abgabevorgangs, an die Auswertungseinheit zu übertragen.

14. Verfahren zum Auswerten einer Medikamenten-Verabreichungsvorrichtung (100) nach einem der vorstehenden Ansprüche 1 bis 12, umfassend den Schritt des Vergleichens einer Temperatur an der Wärmekopplungsfläche (130) mit einer vorgegebenen Temperatur oder einem vorgegebenen Temperaturbereich, wobei die Temperatur vorzugsweise von dem wärmegesteuerten Element abgeleitet wird, z.B. vor, während oder nach dem Verabreichungsvorgang.

## Revendications

1. Dispositif d'administration de médicament (100), le dispositif d'administration de médicament comprenant :
- une surface de couplage thermique (130) agencée et configurée pour transférer de l'énergie thermique,
- un organe à commande thermique (150), connecté de manière thermoconductrice à la surface de couplage thermique, l'organe à commande thermique (150) étant configuré pour fonctionner en réponse à de l'énergie thermique transférée à travers la surface de couplage thermique (130), et le dispositif d'administration de médicament (100) étant configuré pour réaliser une opération d'administration de médicament, l'organe à commande thermique (150) étant agencé et configuré pour se déplacer en réponse à de l'énergie thermique transférée à travers la surface de couplage thermique (130), le déplacement étant utilisé pour le fonctionnement du dispositif d'administration de médicament ;
le dispositif d'administration de médicament (100) comprenant une surface d'activation (124), la surface d'activation étant agencée pour qu'un utilisateur vienne à son contact afin d'amorcer l'opération d'administration de médicament du dispositif d'administration de médicament, et la surface de couplage thermique (130) étant accessible sur la surface d'activation ;
et
des régions entourant latéralement la surface de couplage thermique ou adjacentes à celle-ci étant formées d'un matériau présentant une conductivité thermique inférieure à celle de la surface de couplage thermique.

2. Dispositif d'administration de médicament selon la revendication 1,
l'organe à commande thermique (150) étant un capteur de température électrique, de préférence électronique, ou un interrupteur électrique, de préférence électronique, déclenchable par la température.

3. Dispositif d'administration de médicament selon l'une quelconque des revendications précédentes, l'organe à commande thermique (150) étant un organe bimétallique.

4. Dispositif d'administration de médicament selon l'une quelconque des revendications précédentes, l'organe à commande thermique (150) étant conçu pour fonctionner à une température supérieure ou égale à l'une des valeurs suivantes : 22 °C, 23 °C, 24 °C, 25 °C, 26 °C, 27 °C, 28 °C, 29 °C, 30 °C, 31 °C, 32 °C, 33 °C, 34 °C.

5. Dispositif d'administration de médicament selon l'une quelconque des revendications précédentes, l'organe à commande thermique (150) étant conçu pour fonctionner à une température inférieure ou égale à l'une des valeurs suivantes : 44 °C, 43 °C, 42 °C, 41 °C, 40 °C, 39 °C, 38 °C, 37 °C, 36 °C, 35 °C, 34 °C, 33 °C, 32 °C, 31 °C, 30 °C, 29 °C.

6. Dispositif d'administration de médicament selon l'une quelconque des revendications précédentes,
le dispositif d'administration de médicament (100) comprenant un logement (110) et un récipient (114), situé dans le logement ou relié de manière permanente ou libérable au logement, le récipient étant conçu pour recevoir un contenant de médicament (160) ou le contenant de médicament étant reçu dans le récipient, et le dispositif d'administration de médicament comprenant un mécanisme d'entraînement, lequel est actionnable pour entraîner l'opération d'administration de médicament, le mécanisme d'entraînement comprenant au moins un organe d'entraînement mobile (190) configuré pour être déplacé par rapport au logement afin d'administrer un médicament à partir du contenant de médicament à travers une ouverture de distribution du dispositif d'administration de médicament pendant l'opération d'administration de médicament.

7. Dispositif d'administration de médicament selon la revendication 6,
la surface de couplage thermique (130) étant associée au récipient du dispositif d'administration de médicament (100) de sorte que la surface de couplage thermique est reliée de manière thermoconductrice au contenant de médicament, lorsque le contenant de médicament est agencé dans le récipient.

8. Dispositif d'administration de médicament selon l'une quelconque des revendications précédentes,
le dispositif d'administration de médicament (100) étant configuré de sorte qu'en réponse à de l'énergie thermique fournie à la surface de couplage thermique (130), un verrou mécanique est libéré, le verrou mécanique, lorsqu'il est établi, empêchant le déplacement d'un élément du dispositif et, lorsque le verrou mécanique est libéré, le déplacement de l'élément est autorisé.

9. Dispositif d'administration de médicament selon la revendication 8, l'élément étant un organe de déclenchement pour l'amorçage de l'opération d'administration de médicament, et l'organe de déclenchement étant un bouton de déclenchement ou un protège-aiguille.

10. Dispositif d'administration de médicament selon l'une quelconque des revendications précédentes,
le dispositif d'administration de médicament comprenant une surface d'appui (116), laquelle est agencée pour entrer en contact avec la peau d'un utilisateur ou d'un patient pendant et/ou avant l'opération d'administration de médicament, la surface de couplage thermique (130) étant accessible sur la surface d'appui.

11. Dispositif d'administration de médicament selon l'une quelconque des revendications précédentes,
le dispositif d'administration de médicament (100) comprenant une unité de commande électronique (200), l'organe à commande thermique (150) étant couplé ou apte à être couplé de manière fonctionnelle à l'unité de commande électronique et le dispositif d'administration de médicament étant configuré de sorte que l'unité de commande électronique ne permette l'amorçage de l'opération d'administration de médicament que si une température au niveau de la surface de couplage thermique (130) s'inscrit dans une plage de température prédéterminée.

12. Dispositif d'administration de médicament selon l'une quelconque des revendications précédentes,
le dispositif d'administration de médicament (100) étant configuré pour stocker et/ou transmettre des données sur la température au niveau de la surface de couplage thermique, en particulier lorsque l'opération d'administration de médicament a été amorcée et/ou tandis que l'opération d'administration de médicament est réalisée.

13. Système comprenant le dispositif d'administration de médicament (100) selon l'une quelconque des revendications précédentes et unité d'évaluation (500) intégrée dans le dispositif d'administration de médicament ou apte à être couplée de manière fonctionnelle à celui-ci, le dispositif d'administration de médicament étant configuré pour transférer les données de température indiquant la température au niveau de la surface de couplage thermique, en particulier pendant une opération d'administration, à l'unité d'évaluation.

14. Procédé d'évaluation d'un dispositif d'administration de médicament (100) selon l'une quelconque des revendications précédentes 1 à 12, comprenant l'étape de comparaison d'une température au niveau de la surface de couplage thermique (130) avec une température ou une plage de température prédéterminée, la température étant de préférence dérivée de l'organe à commande thermique, par exemple avant, pendant ou après l'opération d'administration.
